Europäisches Patentamt

European Patent Office

Office européen des brevets

(11) Veröffentlichungsnummer: **0 374 088**

**A1**

(12) **EUROPÄISCHE PATENTANMELDUNG**

(21) Anmeldenummer: **89810755.2**

(22) Anmeldetag: **04.10.89**

(51) Int. Cl.⁵: **A61B 17/58, A61F 2/00**

(30) Priorität: **16.12.88 CH 4654/88**

(43) Veröffentlichungstag der Anmeldung:
**20.06.90 Patentblatt 90/25**

(84) Benannte Vertragsstaaten:
**AT DE FR GB IT**

(71) Anmelder: **GEBRÜDER SULZER
AKTIENGESELLSCHAFT
Zürcherstrasse 9
CH-8401 Winterthur(CH)**

Anmelder: **Protek AG
Stadtbachstrasse 64
CH-3001 Bern(CH)**

(72) Erfinder: **Müller, Maurice E. Prof. Dr.-med.
Melchenbühlweg 9
CH-3006 Bern(CH)**
Erfinder: **Spotorno, Lorenzo Dr.-med.
Ospedale Riunitit
I-17024 Finale Ligure(IT)**
Erfinder: **Frey, Otto Dr.
Wallrütistrasse 56
CH-8400 Winterthur(CH)**

(54) Knochenschraube.

(57) Die ein- oder mehrgängige Knochenschraube ist korkenzieherartig ausgebildet, d.h. ihre Gewindegänge (4) sind nicht auf einen massiven Kern aufgebracht, sondern "umschlingen" einen zylindrischen Hohlraum (5).

Die neue Form des Gewindes (4) ermöglicht eine weitgehende Anpassung der Biegeelastizität der Schraube an die Biegeelastizität des Knochens.

Fig. 1

EP 0 374 088 A1

# Knochenschraube

Die Erfindung betrifft eine Knochenschraube zum Fixieren von Implantaten in spongiosem Knochengewebe.

Knochenschrauben zum Fixieren von Implantaten sind seit langem bekannt; sie sind bisher so ausgeführt worden, dass ein ein-oder mehrgängiges Gewinde - wie bei einer Holzschraube - von einem massiven Kern getragen wird. Besonders für Biegebelastungen sind diese Knochenschrauben relativ zu dem sie umgebenden Gewebe ein unelastischer Fremdkörper, der den elastischen Verformungen des Knochens Widerstand entgegensetzt und damit die Elastizität des Knochens beeinträchtigt.

Aufgabe der Erfindung ist es daher, eine Knochenschraube zu schaffen, deren Elastizität an die des Knochengewebes besser angepasst ist als bei den bisherigen Konstruktionen. Mit der Erfindung wird diese Aufgabe dadurch gelöst, dass der an einen Schraubenkopf angesetzte Gewindekörper als korkenzieherartiger Hohlzylinder ausgebildet ist.

Beim Einschrauben der neuen Schraube bleibt in Innern ihres Hohlzylinders ein "Kern" von lebendem Knochengewebe erhalten, das nach kurzer Zeit mit dem die Schraube aussen umgebenden Gewebe wieder zusammenwächst und so lebensfähig erhalten werden kann.

Da dieser Knochenkern, der die Elastizität der Schraube gegen Biegung im wesentlichen bestimmt, die Elastizität des Knochens hat, ist auch die Elastizität der Schraube vor allem bei Biegebelastungen derjenigen des "ungestörten" Knochens weitgehend angenähert.

Um eine gute "Ernährung" des Knochenkerns im Hohlzylinder der Schraube sicherzustellen, ist es vorteilhaft, wenn die Steigung des Gewindes mindestens gleich der Querschnittsabmessung parallel zur Schraubenachse ist.

Praktisch keine Kräfte in radialer Richtung werden von der Schraube ausgelöst, wenn der Querschnitt der im Mantel des Hohlzylinders verlaufenden Gewindegänge rechteckig oder quadratisch ist; dabei hat es sich für die Festigkeit und Stabilität des Gewindes als vorteilhaft erwiesen, wenn die Querschnittsabmessung senkrecht zur Schraubenachse höchstens das 2-fache derjenigen parallel dazu beträgt.

Unter Inkaufnahme relativ geringer Kraftwirkungen in radialer Richtung kann das Einschrauben der Schraube in den Knochen erleichtert werden, wenn der Querschnitt der im Mantel des Hohlzylinders verlaufenden Gewindegänge aus einem Rechteck oder Quadrat und einem Dreieck bzw. einem Kreissektor oder Halbkreis zusammengesetzt ist, wobei Dreieck, Halbkreis oder Kreissektor nach aussen gerichtet sind.

Schliesslich ist es auch möglich, die Schraube 2- oder mehrgängig auszubilden, womit die Führung beim Einschrauben der Schraube verbessert und die Umdrehungszahl zum vollständigen Einschrauben verkleinert wird.

Im folgenden wird die Erfindung anhand von Ausführungsbeispielen im Zusammenhang mit der Zeichnung näher erläutert.

Fig. 1 ist eine Ansicht der neuen Knochenschraube, deren Gewindegänge teilweise im Schnitt dargestellt sind;

Fig. 2 und 3 sind Ansichten von Fig. 1 von unten bzw. von oben;

Fig. 4, 5, 6 geben verschiedene Querschnitte für die Gewindegänge wieder, während

Fig. 7 in gleicher Darstellung wie Fig. 1 eine zweite Ausführungsform zeigt, die zweigängig ausgeführt ist.

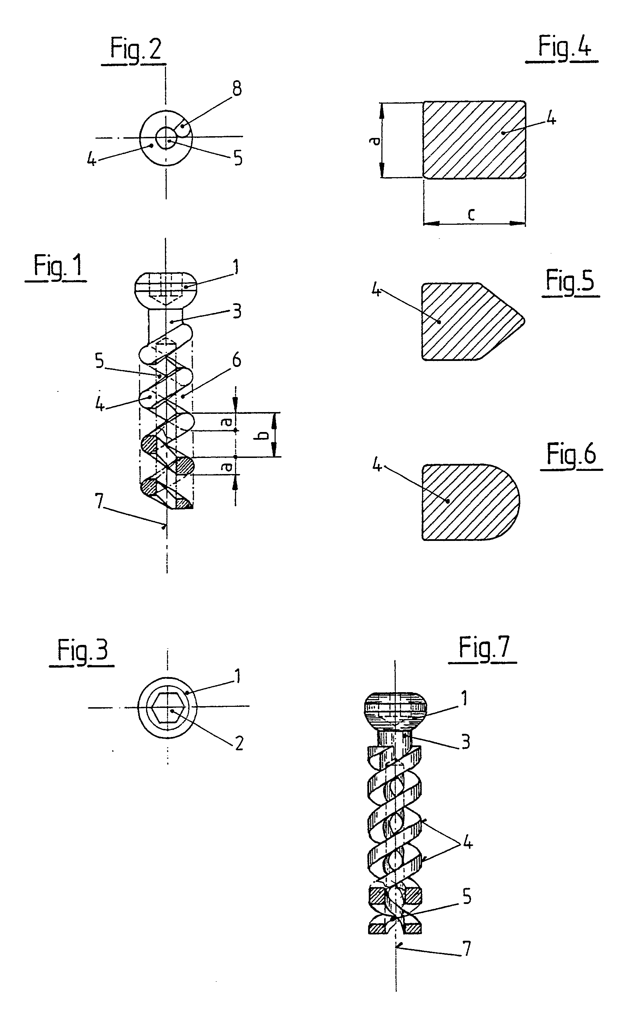

Ein Schraubenkopf 1 (Fig. 1), der mit einem Innensechskant 2 (Fig. 3) für den Einsatz eines Werkzeuges versehen ist, geht in einen kurzen massiven Hals 3 über. An diesen schliesst ein in Fig. 1 eingängiges Gewinde 4 an, welches korkenzieherartig als Mantel 6 eines Hohlzylinders einen Hohlraum 5 (Fig. 2) umgibt. In Fig. 1 ist der Querschnitt des einzigen Gewindeganges 4 rechteckig, wobei die äusseren Kanten abgerundet sind.

Die Steigung b des Gewindes 4 ist gleich oder grösser als die doppelten Querschnittsabmessungen 2.a in Richtung der Schraubenachse 7.

An seinem freien Ende 8 ist das Gewinde 4 zungenartig abgeflacht, um das Einschrauben in den Knochen zu erleichtern.

Wie bereits erwähnt, sind rechteckige oder quadratische Querschnitte der Gewindegänge 4 zu bevorzugen, da sie praktisch keine Kraftkomponenten in radialer Richtung auf den Knochen ausüben. Einen geeigneten rechteckigen Querschnitt zeigt Fig. 2. Zur Vermeidung von Spitzenbelastungen im Knochen sind die Kanten des Querschnittes gerundet. Um dem Gewinde eine ausreichende Stabilität zu geben, sollte das Verhältnis der Rechteckabmessungen c senkrecht zu denjenigen a parallel zur Schraubenachse 7 höchstens 2:1 betragen; in dem gezeigten Beispiel ist dieses Verhältnis $c / a = 1.35$.

In Fig. 5 setzt sich der Querschnitt der Gewindegänge a aus einem Rechteck und einem Dreieck zusammen. Eine derartige Form erzeugt zwar Kraftwirkungen auf den Knochen in radialer Richtung, diese werden jedoch in Kauf genommen für den Vorteil einer gegenüber einem quadratischen oder rechteckigen Querschnitt erleichterten Einschraubbarkeit; da das Dreieck gleichschenkelig ausgebildet ist, heben sich an den Dreiecksflächen, beispielsweise beim Einschrauben, auftretende Momente auf.

In seiner Wirkung hinsichtlich radialer Kraftkomponenten und einer erleichterten Einschraubbarkeit liegt der Querschnitt nach Fig. 6, der sich aus einem Rechteck und einem Kreissektor, vorteilhafterweise einem Halbkreis, zusammensetzt, zwischen den Formen nach Fig. 4 und 5. Die im Zusammenhang mit Fig. 4 erläuterte Stabilitätsbedingung ist auch bei den Querschnittsformen nach Fig. 5 und 6 eingehalten worden.

Die Knochenschraube nach Fig. 7 unterscheidet sich von derjenigen nach Fig. 1 dadurch, dass das Gewinde 4 zweigängig ausgeführt ist; beide Gewindegänge der Schraube nach Fig. 7 haben einen quadratischen Querschnitt. Eine solche zweigängige Schraube hat den Vorteil, dass sie sich beim Einschrauben selbst zentriert und dass sie zum Setzen nur die halbe Umdrehungszahl benötigt.

**Ansprüche**

1. Knochenschraube zum Fixieren von Implantaten in spongiosem Knochengewebe, dadurch gekennzeichnet, dass der an einen Schraubenkopf (1) angesetzte Gewindekörper(4) als korkenzieherartiger Hohlzylinder ausgebildet ist.

2. Knochenschraube nach Anspruch 1, dadurch gekennzeichnet, dass der Querschnitt der im Mantel (6) des Hohlzylinders verlaufenden Gewindegänge (4) rechteckig oder quadratisch ist.

3. Knochenschraube nach Anspruch 1 oder 2, dadurch gekennzeichnet, dass der Querschnitt der im Mantel(6) des Hohlzylinders verlaufenden Gewindgänge (4) aus einem Rechteck oder Quadrat und einem Dreieck zusammengesetzt ist, wobei das Dreieck nach aussen gerichtet ist.

4. Knochenschraube nach einem der Ansprüche 1 bis 3, dadurch gekennzeichnet, dass der Querschnitt der im Mantel (6) des Hohlzylinders verlaufenden Gewindgänge (4) aus einem Rechteck oder Quadrat und einem Kreissektor oder Halbkreis zusammengesetzt ist, wobei der Kreissektor oder Halbkreis nach aussen gerichtet ist.

5. Knochenschraube nach einem der Ansprüche 2 bis 4, dadurch gekennzeichnet, dass die Querschnittsabmessung (c) senkrecht zur Schraubenachse (7) höchstens das Zwei-fache derjenigen (a) parallel dazu beträgt.

6. Knochenschraube nach einem der Ansprüche 1 bis 5, dadurch gekennzeichnet, dass die Steigung (b) des Gewindes (4) mindestens gleich der doppelten Querschnittsabmessung (2.a) parallel zur Schraubenachse (7) ist.

7. Knochenschraube nach einem der Ansprüche 1 bis 6, dadurch gekennzeichnet, dass das Gewinde (4) mindestens zweigängig ausgebildet ist.

89010755.2

Fig.2

8

4          5

Fig.4

d

c

4

Fig.1

1

3

5

6

4

a

b

a

7

Fig.5

4

Fig.6

4

Fig.3

1

2

Fig.7

1

3

4

5

7

## EINSCHLÄGIGE DOKUMENTE

EP 89810755.2

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | EP - A2 - 0 172 130 <br> (ME CRON) <br> * Claim 1; fig. 1 * <br> -- | 1 | A 61 B 17/58 <br> A 61 F  2/00 |
| A | DE - A1 - 3 538 238 <br> (FISCHER) <br> * Claims 1-3; fig. 1 * <br> ---- | 1 | |

| RECHERCHIERTE SACHGEBIETE (Int Cl⁵) |
|---|
| A 61 B <br> A 61 F |

Der vorliegende Recherchenbericht wurde für alle Patentansprüche erstellt.

| Recherchenort | Abschlußdatum der Recherche | Prüfer |
|---|---|---|
| WIEN | 28-03-1990 | MIHATSEK |